# FASCICULE DE BREVET EUROPEEN

(11) **EP 0 648 731 B1**
(45) Date de publication et mention de la délivrance du brevet: **07.01.1998**
(21) Numéro de dépôt: 94420263.9
(22) Date de dépôt: 28.09.1994
(51) Int. Cl.: C07C 57/03, C07C 51/14

(54) **Procédé d'hydroxycarbonylation du butadiène**
Verfahren zur Hydroxycarbonylierung von Butadien
Process for hydroxycarbonylation of butadiene

(30) Priorité: 19.10.1993 FR 9312666
(43) Date de publication de la demande: 19.04.1995
(73) Titulaire: RHONE-POULENC FIBER & RESIN INTERMEDIATES, 92405 Courbevoie (FR)
(72) Inventeur: Denis, Philippe, F-69150 Decines (FR); Patois, Carl, F-69003 Lyon (FR); Perron, Robert, F-69390 Charly (FR)
(74) Mandataire: Vignally, Noel

(56) Documents cités:
- FR-A- 1 402 383

## Description

La présente invention concerne l'hydroxycarbonylation du butadiène et/ou de ses dérivés, en acides penténoïques, par réaction avec le monoxyde de carbone et l'eau.

Une des voies possibles d'accès à l'acide adipique, qui est l'un des deux constituants de base des polyamides 6.6, est la double carbonylation du butadiène ou de ses dérivés.

Bien qu'il puisse être imaginé de réaliser en une seule étape les deux hydroxycarbonylations menant du butadiène à l'acide adipique, il s'avère en pratique que les deux réactions doivent être conduites successivement, si l'on veut obtenir des sélectivités suffisamment élevées pour pouvoir envisager un procédé industriel économiquement viable.

Le brevet US-A-3 509 209 décrit l'hydroxycarbonylation de diverses oléfines, dont le butadiène, par le monoxyde de carbone et l'eau, en présence d'acide chlorhydrique ou bromhydrique et d'un catalyseur contenant du palladium, à une température de 15°C à 300°C et sous une pression de 1 à 1000 bars et de préférence de 10 à 200 bars.

Dans les conditions décrites, on observe que les rendements en acides penténoïques sont très faibles et qu'en réalité très souvent le produit obtenu est la valérolactone.

Le brevet FR-A-2 529 885 a proposé un procédé de préparation d'acides béta-gamma-insaturés tels que les acides penténoïques, par carbonylation d'un diène conjugué (butadiène plus particulièrement) en présence d'eau, d'un hydracide halogéné, d'un catalyseur à base de palladium et d'un sel d'onium quaternaire d'un élément choisi parmi l'azote, le phosphore et l'arsenic.

Ce procédé donne de bons résultats, mais il nécessite l'emploi d'une quantité relativement importante d'un sel d'onium quaternaire, composé onéreux et dont la présence est de nature à compliquer le traitement des mélanges en fin de réaction.

Il a maintenant été trouvé que l'on peut réaliser l'hydroxycarbonylation du butadiène et de ses dérivés, avec une très bonne sélectivité en produits valorisables, c'est-à-dire notamment en acides penténoïques, sans pour autant opérer en présence d'un sel d'onium quaternaire.

Plus précisément la présente invention consiste en un procédé d'hydroxycarbonylation du butadiène et de ses dérivés, par le monoxyde de carbone et l'eau, à une pression supérieure à la pression atmosphérique et en présence d'un catalyseur au palladium soluble dans le milieu réactionnel, caractérisé en ce que :
- l'on opère en présence de chlorure de crotyle, à raison d'au moins deux moles par mole de palladium,
- le palladium se trouve au moins en partie sous forme de complexe pi-crotyl,
- l'eau présente dans le milieu réactionnel représente une quantité inférieure ou égale à 20 % en poids par rapport au poids du mélange réactionnel.

Par dérivé du butadiène, on entend dans le présent texte notamment les buténols allyliques comme le 3-butène-2-ol, le 2-butène-1-ol et leurs mélanges, les composés d'addition du chlorure d'hydrogène sur le butadiène (chlorobutènes) dont le principal est le chlorure de crotyle. Dans le présent procédé, on peut mettre en oeuvre le butadiène, un ou plusieurs de ses dérivés ou les mélanges du butadiène avec un ou plusieurs de ses dérivés. Le butadiène ou les mélanges contenant une partie majoritaire de butadiène représentent cependant les substrats mis en oeuvre de préférence.

Le catalyseur complexe pi-crotyl-palladium peut être introduit dans le milieu réactionnel ou être formé in situ à partir des halogénures de Pd, plus particulièrement le chlorure, des carboxylates de Pd, notamment l'acétate ou encore à partir de palladium métallique finement divisé.

La quantité de catalyseur pi-crotyl-palladium utilisée dans le procédé peut varier dans de larges limites. Généralement on met en oeuvre de 10⁻⁵ mole à 0,2 mole de Pd par mole de butadiène ou de dérivé du butadiène engagé dans la réaction et de préférence de 10⁻⁴ mole à 0,1 mole par mole.

Outre le catalyseur pi-crotyl-palladium peut se trouver également dans le milieu réactionnel du palladium sous une autre forme moins active (par exemple du Pd métallique ou du chlorure de Pd) en quantité variable. Dans un procédé industriel, il est cependant préférable que tout ou pratiquement tout le palladium soit sous une forme active et soluble dans le milieu, comme le pi-crotyl-palladium, éventuellement avec du chlorure de palladium.

Le complexe pi-crotyl-palladium peut être préparé par exemple en faisant réagir un sel de palladium, comme le chlorure de palladium, avec du chlorure de crotyle, dans un solvant pouvant être constitué par un mélange eau/méthanol. Le mélange est agité, généralement à température ambiante, avantageusement sous léger courant de monoxyde de carbone. Le complexe pi-crotyl-palladium précipite et après une éventuelle phase de dégazage, le mélange est versé dans de l'eau, puis est extrait à l'aide d'un solvant organique adapté, tel que par exemple du chloroforme. Le complexe est ensuite isolé de la solution organique par évaporation du solvant.

Le promoteur chlorure de crotyle peut être introduit dans le mélange réactionnel ou être formé in situ, à partir de butadiène et/ou de 2-butène-1-ol et d'acide chlorhydrique.

Il représente, en mole par mole, de préférence de 5 à 10 fois la quantité de palladium, bien qu'il puisse être présent en plus grandes proportions, puisqu'il peut constituer tout ou partie du substrat à hydroxycarbonyler.

Globalement il est préférable d'avoir dans le milieu réactionnel un rapport molaire Cl/Pd inférieur ou égal à 100 et de préférence inférieur ou égal à 20, car des rapports élevés ont une influence néfaste sur la cinétique de la réaction.

Comme indiqué précédemment, la concentration en eau dans le mélange réactionnel doit être maintenue à une valeur égale ou inférieure à 20 % en poids par rapport au poids dudit mélange. En effet la concentration en eau a un effet défavorable sur la cinétique de la réaction. De préférence cette concentration en eau sera maintenue à une valeur égale ou inférieure à 8 % en poids par poids et encore plus préférablement à une valeur égale ou inférieure à 5 %.

L'eau étant un réactif indispensable à la réaction d'hydroxycarbonylation, une variante interessante du procédé de l'invention consiste à injecter cette eau au fur et à mesure de l'avancement de la réaction, ce qui permet de maintenir sa concentration dans le mélange réactionnel à une valeur très faible, tout en permettant à la réaction de s'effectuer.

Bien que la présence d'un tiers-solvant ne soit pas exclue, la réaction est généralement conduite sans solvant autre que les réactifs eux-mêmes ou les produits de la réaction. Il peut également être favorable d'introduire dès le début de la réaction d'hydroxycarbonylation un acide penténoïque, et plus particulièrement l'acide pentène-3-oïque, afin de minimiser les réactions secondaires.

Dans le cadre d'une mise en oeuvre industrielle du procédé, des recyclages du catalyseur, du promoteur, du butadiène n'ayant pas réagi, peuvent conduire à l'introduction dans le milieu réactionnel de quantités plus ou moins importantes d'autres composés, notamment de sous-produits formés lors de la réaction d'hydroxycarbonylation. Ainsi, on peut avoir dans le mélange réactionnel par exemple des butènes, de la gamma-valérolactone, de l'acide valérique, de l'acide adipique, de l'acide méthyl-2 glutarique, de l'acide éthyl-2 succinique, de l'acide méthyl-2-butanoïque, des acides méthyl-2-buténoïques. Compte tenu des impératifs d'une possible mise en oeuvre en continu du procédé, les quantités présentes de ces composés peuvent atteindre jusqu'à 90 % en poids du mélange réactionnel engagé dans la réaction d'hydroxycarbonylation.

La concentration en butadiène est un paramètre important de la réaction, notamment pour ce qui concerne la stabilité du catalyseur au palladium, c'est-à-dire essentiellement son maintien en solution dans le mélange réactionnel. Il a ainsi été observé qu'il n'est pas favorable d'avoir moins de 0,2 % en poids de butadiène par rapport au poids total du mélange réactionnel. De préférence, lorsque l'on opère en discontinu, la conversion du butadiène ou de ses dérivés sera limitée de manière à ce que le mélange réactionnel renferme au moins 0,5 % en poids dudit butadiène ou de ses dérivés.

La concentration en butadiène sera également de préférence maintenue à une valeur égale ou inférieure à 50 % en poids par poids du mélange réactionnel et encore plus préférentiellement à une valeur égale ou inférieure à 30 %, lorsque l'on opère en procédé discontinu et à une valeur égale ou inférieure à 10 % lorsque l'on opère en procédé continu.

Alors que le catalyseur au palladium tend à précipiter dans une proportion importante sous forme de palladium métallique insoluble, lorsque l'on réalise l'hydroxycarbonylation du butadiène dans des conditions différentes de celles du présent procédé, on observe au contraire qu'avec les conditions revendiquées le catalyseur conserve une remarquable stabilité.

Au plan industriel, il est très avantageux de ne véhiculer dans l'appareillage que des phases liquides et donc d'éviter le plus possible la présence de solides en suspension. C'est notamment ce que permet le présent procédé.

La réaction d'hydroxycarbonylation peut être conduite à une température généralement située entre 60°C et 230°C et de préférence entre 90°C et 200°C et sous une pression en température de 50 à 500 bars et de préférence de 100 à 300 bars.

La pression.partielle de monoxyde de carbone, mesurée à 25°C, est de 25 bars à 440 bars et de préférence de 55 bars à 240 bars.

Comme cela a été indiqué, le procédé de l'invention peut être mis en oeuvre de manière continue ou discontinue. Selon la mise en oeuvre choisie, il y aura donc lieu d'adapter les différentes conditions opératoires définies précédemment.
Les exemples qui suivent illustrent l'invention.

### EXEMPLE 1.

### 1) Préparation du complexe chlorure de pi-crotyl-Pd.

Dans un ballon en verre de 150 cm³, on charge successivement 5,04 g de PdCl₂, 3,37 g de NaCl, 50 cm³ de méthanol, 15 cm³ d'eau, 8,03 g de chlorure de crotyle et à nouveau 20 cm³ de méthanol.

On agite le mélange hétérogène qui devient progressivement marron foncé et trouble. On traite ensuite la solution sous agitation par un léger courant de monoxyde de carbone (bulle à bulle) pendant une heure. Le mélange s'éclaircit et un précipité jaune apparaît. L'agitation et le courant de CO sont arrêtés, la solution est abandonnée pendant une heure au repos, puis elle est versée dans 300 cm³ d'eau, extraite par 5 fois 50 cm³ de chloroforme. La phase organique résultante jaune paille est lavée par 2 fois 100 cm³ d'eau, séchée sur sulfate disodique pendant une nuit, puis le solvant est évaporé. On récupère ainsi 3,35 g d'un solide jaune pâle ayant une pureté supérieure à 94 % (dosage par Résonance Magnétique Nucléaire : RMN).

### 2) Hydroxycarbonylation du butadiène.

On charge successivement les réactifs suivants dans une ampoule de verre de 50 cm³:

| | |
|---|---|
| - chlorure de pi-crotyl Pd: | 0,9 mmol |
| - chlorure de crotyle: | 6,2 mmol |
| - H₂O: | 100 mmol |
| - acide pentène-3 oïque: | 20 g (200 mmol) |
| - butadiène: | 100 mmol |

Le butadiène est introduit en dernier lieu, par condensation (paroi froide à - 78°C) à partir d'un sas.

L'ampoule est placée dans un autoclave de 125 cm³; l'autoclave est placé dans un four à agitation par secousses, raccordé à un système d'alimentation en gaz à haute pression et on établit une pression de 100 bar de CO à température ambiante.

La température est ensuite portée à 140°C sous agitation sur une période de 25 min. A cette température, la pression dans l'autoclave est portée à 200 bar par introduction de CO et elle est maintenue constante à cette valeur grâce à une réserve de CO sous pression pendant 30 min.

L'agitation est alors arrêtée, l'autoclave refroidi, dégazé et les gaz ainsi que la solution obtenue sont analysés par chromatographie en phase gazeuse (CPG).

On a obtenu les résultats suivants:

| | |
|---|---|
| - taux de transformation du butadiène (TTBD): | 92 % |
| - rendement (RT) en acide pentène-3 oïque (P3) par rapport au butadiène transformé: | 89,5 % |
| - RT en acide adipique (A1): | 0,1 % |
| - RT en acide méthyl-2 glutarique (A2): | 4,1 % |
| - RT en acide éthyl-2 succinique (A3): | 1,8 % |
| - RT en gamma-valérolactone (VAL): | 0,7 % |
| - RT en acide pentène-2 oïque (P2): | 0,3 % |
| - RT en butènes (C4): | 0,6 % |
| - RT en acides méthyl-buténoïques et méthyl-butanoïque (MB): | 2,4 % |

En fin d'essai, tout le palladium chargé demeure en solution.

La productivité de l'essai est de 500 g P3/litre de réacteur/heure.

### EXEMPLE 2

On répète la partie 2) de l'exemple 1, avec les mêmes charges exceptée la quantité d'eau qui est de 50 mmol au lieu de 100 mmol.

Les conditions de température et de pression sont les mêmes que pour l'exemple 1.

On obtient les mêmes rendements dans les différents produits formés, mais la productivité de l'essai est de 1200 g P3/litre de réacteur/heure.

### EXEMPLE 3

On répète la partie 2) de l'exemple 1, avec les charges suivantes :

| | |
|---|---|
| - acétate de Pd: | 0,9 mmol |
| - chlorure de crotyle: | 7,2 mmol |
| - H₂O: | 100 mmol |
| - acide pentène-3 oïque: | 20 g (200 mmol) |
| - butadiène: | 100 mmol |

L'appareillage et le mode opératoire sont les mêmes que dans l'exemple 1.

Les conditions opératoires sont : 140°C ; 200 bar en température ; 30 min en température et pression.

On a obtenu les résultats suivants:

| | |
|---|---|
| - TT du butadiène: | 87 % |
| - RT en acide pentène-3 oïque (P3): | 91 % |
| - RT en acide adipique (A1): | 0,1 % |
| - RT en acide méthyl-2 glutarique (A2): | 3 % |
| - RT en acide éthyl-2 succinique (A3): | 2 % |
| - RT en gamma-valérolactone (VAL): | 0,8 % |
| - RT en acide pentène-2 oïque (P2): | 0,2 % |
| - RT en butènes (C4): | 0,3 % |
| - RT en acides méthyl-buténoïques et méthyl-butanoïque (MB): | 2 % |

En fin d'essai, 97 % du palladium chargé demeurent en solution.

La productivité de l'essai est de 500 g P3/litre de réacteur/heure.

### EXEMPLES 4 A 8

On répète la partie 2) de l'exemple 1, avec les charges suivantes :

| | |
|---|---|
| - chlorure de pi-crotyl-Pd (préparé dans l'exemple 1): | 0,9 mmol |
| - chlorure de crotyle: | 6,2 mmol |
| - H₂O: | 100 mmol |
| - acide pentène-3 oïque: | 20 g (200 mmol) |
| - butadiène: | 100 mmol |

L'appareillage et le mode opératoire sont les mêmes que dans l'exemple 1.

Les conditions opératoires sont : 140°C ; 200 bar en température ; 25 min en température et pression.

La durée de 25 min est choisie de manière à avoir un TT du butadiène d'environ 80 %. Après une première opération d'hydroxycarbonylation, l'agitation est arrêtée, l'autoclave est refroidi, dégazé et la solution homogène obtenue est mise sous pression réduite, afin de distiller l'acide pentène-3 oïque formé (entre 5 et 7 g).

Le culot de distillation est toujours homogène et il est réengagé dans l'ampoule de verre, avec de nouvelles charges de chlorure de crotyle (6,2 mmol), d'eau (100 mmol) et de butadiène (100 mmol).

On réalise ainsi 5 essais successifs d'hydroxycarbonylation du butadiène dans les mêmes conditions opératoires.

Les distillats obtenus ainsi que le culot final sont analysés par CPG.

On a obtenu les résultats suivants (sur l'ensemble des 5 essais):

| | |
|---|---|
| - TT du butadiène: | 80 % |
| - RT en acide pentène-3 oïque (P3): | 93 % |
| - RT en acide adipique (A1): | 0 % |
| - RT en acide méthyl-2 glutarique (A2): | 2,6 % |
| - RT en acide éthyl-2 succinique (A3): | 0,9 % |
| - RT en gamma-valérolactone (VAL): | 0,8 % |
| - RT en acide pentène-2 oïque (P2): | 0 % |
| - RT en acides méthyl-buténoïques et méthyl-butanoïque (MB): | 1,7 % |

En fin d'essai, tout le palladium chargé demeure en solution.

La productivité de l'essai est de 415 g P3/litre de réacteur/heure.

### EXEMPLES 9 et 10

On répète la partie 2) de l'exemple 1, avec les charges suivantes :

| | |
|---|---|
| - chlorure de pi-crotyle-Pd: | 0,9 mmol |
| - chlorure de crotyle: | 7,1 mmol |
| - H₂O: | 120 mmol |
| - acide pentène-3 oïque: | 20 g (200 mmol) |
| - butadiène: | 100 mmol |

Les réactifs sont chargés directement dans l'autoclave (en alliage nickelmolybdène de marque Hastelloy B2) et le mode opératoire est le même que dans l'exemple 1.

Les conditions opératoires sont : 140°C ; 200 bar en température ; 20 min en température et pression.

On a obtenu les résultats suivants:

| | |
|---|---|
| - TT du butadiène: | 81 % |
| - RT en acide pentène-3 oïque (P3): | 92 % |
| - RT en acide adipique (A1): | 0,1 % |
| - RT en acide méthyl-2 glutarique (A2): | 3,6 % |
| - RT en acide éthyl-2 succinique (A3): | 2,2 % |
| - RT en gamma-valérolactone (VAL): | 1,8 % |
| - RT en acide pentène-2 oïque (P2): | 0,1 % |
| - RT en butènes (C4): | 0,1 % |
| - RT en acides méthyl-buténoïques et méthyl-butanoïque (MB): | 2,4 % |

En fin d'essai, tout le palladium chargé demeure en solution.

La concentration finale en butadiène est de 2 % en poids du mélange réactionnel.

La productivité est de 800 g P3/litre de réacteur/heure.

L'exemple 9 est répété avec les mêmes charges et les mêmes conditions opératoires, mais il est maintenu pendant 40 min en température et pression au lieu de 20 min (exemple 10).

Les rendements obtenus sont les mêmes que dans l'exemple 9, mais la concentration finale en butadiène est de 0,35 % en poids dans le mélange réactionnel.

On observe que 20 % du catalyseur chargé ont précipité sous forme de Pd métallique.

### EXEMPLE 11

On répète la partie 2) de l'exemple 1, avec les charges suivantes :

| | |
|---|---|
| - chlorure de pi-crotyle-Pd: | 0,9 mmol |
| - chlorure de crotyle: | 112 mmol |
| - H₂O: | 100 mmol |
| - acide pentène-3 oïque: | 20 g (200 mmol) |

L'appareillage et le mode opératoire sont les mêmes que dans l'exemple 1.

Les conditions opératoires sont : 140°C ; 200 bar en température ; 40 min en température et pression.

On a obtenu les résultats suivants:

| | |
|---|---|
| - TT du chlorure de crotyle: | 90 % |
| - RT en acide pentène-3 oïque (P3): | 26 % |
| - RT en acide adipique (A1): | 0,1 % |
| - RT en acide méthyl-2 glutarique (A2): | 0,1 % |
| - RT en acide éthyl-2 succinique (A3): | 6,0 % |
| - RT en gamma-valérolactone (VAL): | 17,0 % |
| - RT en acide pentène-2 oïque (P2): | 0,2 % |
| - RT en acides méthyl-buténoïques et méthyl-butanoïque (MB): | 2,0 % |

La productivité est de 130 g P3/litre de réacteur/heure.

### ESSAI COMPARATIF 1 (avec une forte concentration en eau)

On répète la partie 2) de l'exemple 1, avec les charges suivantes :

| | |
|---|---|
| - chlorure de pi-crotyle-Pd: | 0,9 mmol |
| - chlorure de crotyle: | 7,1 mmol |
| - H₂O: | 500 mmol |
| - acide pentène-3 oïque: | 15 g (150 mmol) |
| - butadiène: | 100 mmol |

L'appareillage et le mode opératoire sont les mêmes que dans l'exemple 1.

Les conditions opératoires sont : 140°C ; 200 bar en température ; 103 min en température et pression.

On a obtenu les résultats suivants:

| | |
|---|---|
| - TT du butadiène: | 72 % |
| - RT en acide pentène-3 oïque (P3): | 79 % |
| - RT en acide adipique (A1): | 0,4 % |
| - RT en acide méthyl-2 glutarique (A2): | 2 % |
| - RT en acide éthyl-2 succinique (A3): | 0,3 % |
| - RT en gamma-valérolactone (VAL): | 13 % |
| - RT en butènes (C4): | 1 % |
| - RT en acides méthyl-buténoïques et méthyl-butanoïque (MB): | 5 % |

La productivité est de 87 g P3/litre de réacteur/heure.

En fin d'essai, bien que la concentration en butadiène soit encore de 2 %, on observe qu'environ 25 % du catalyseur chargé ont précipité sous forme de Pd métallique.

En outre, le rendement en acide pentène-3 oïque n'est que de 79 % et la productivité de 87 g P3/l/h.

### EXEMPLE 12

On répète la partie 2) de l'exemple 1, avec les charges suivantes :

| | |
|---|---|
| - chlorure de pi-crotyle-Pd: | 0,9 mmol |
| - chlorure de crotyle: | 7,1 mmol |
| - H₂O: | 100 mmol |
| - acide méthyl-2 glutarique: | 15 g (103 mmol) |
| - acide éthyl-2 succinique: | 5 g (34 mmol) |
| - butadiène: | 100 mmol |

L'appareillage et le mode opératoire sont les mêmes que dans l'exemple 1.

Les conditions opératoires sont : 140°C ; 200 bar en température ; 70 min en température et pression.

On a obtenu les résultats suivants:

| | |
|---|---|
| - TT du butadiène: | 78 % |
| - RT en acide pentène-3 oïque (P3): | 95 % |
| - RT en gamma-valérolactone (VAL): | 0,6 % |
| - RT en butènes (C4): | 0,8 % |
| - RT en acides méthyl-buténoïques et méthyl-butanoïque (MB): | 4 % |

Les diacides formés, représentant de très faibles quantités par rapport aux quantités engagées au début de l'essai, n'ont pas été dosés.

En fin d'essai, tout le palladium chargé demeure en solution.

La productivité est de 164 g P3/litre de réacteur/heure.

## Revendications

1. Procédé d'hydroxycarbonylation du butadiène, ou de l'un de ses dérivés, par le monoxyde de carbone et l'eau, à une pression supérieure à la pression atmosphérique et en présence d'un catalyseur au palladium soluble dans le milieu réactionnel, caractérisé en ce que :
- l'on opère en présence de chlorure de crotyle, à raison d'au moins deux moles par mole de palladium,
- le palladium se trouve au moins en partie sous forme de complexe pi-crotyl,
- l'eau présente dans le milieu réactionnel représente une quantité inférieure ou égale à 20 % en poids par rapport au poids du mélange réactionnel.

2. Procédé selon la revendication1, caractérisé en ce que la pression totale en température est de 50 bars à 500 bars et de préférence de 100 bars à 300 bars.

3. Procédé selon l'une des revendications 1 ou 2, caractérisé en ce que la pression partielle de monoxyde de carbone mesurée à 25°C est de 25 bars à 440 bars et de préférence de 55 bars à 240 bars.

4. Procédé selon l'une des revendications 1 à 3, caractérisé en ce que la quantité de palladium représente de 10⁻⁵ mole à 0,2 mole par mole de butadiène engagé et de préférence de 10⁻⁴ mole à 0,1 mole par mole.

5. Procédé selon l'une des revendications 1 à 4, caractérisé en ce que le chlorure de crotyle représente en moles de 5 à 10 fois la quantité molaire de palladium dans le milieu réactionnel.

6. Procédé selon l'une des revendications 1 à 5, caractérisé en ce que la concentration en butadiène est d'au moins 0,2 % en poids par rapport au poids du milieu réactionnel et de préférence d'au moins 0,5 %.

7. Procédé selon l'une des revendications 1 à 6, caractérisé en ce que la concentration en butadiène est égale ou inférieure à 50 % en poids par rapport au poids du milieu réactionnel et de préférence égale ou inférieure à 30 % lorsque le procédé est mis en oeuvre de manière discontinue.

8. Procédé selon l'une des revendications 1 à 6, caractérisé en ce que la concentration en butadiène est égale ou inférieure à 10 % en poids par rapport au poids du milieu réactionnel lorsque le procédé est mis en oeuvre de manière continue.

9. Procédé selon l'une des revendications 1 à 8, caractérisé en ce que la réaction d'hydroxycarbonylation est conduite dans l'acide pentène-3-oïque.

10. Procédé selon l'une des revendications 1 à 9, caractérisé en ce que la réaction d'hydroxycarbonylation est conduite à une température de 60°C à 230°C et de préférence de 90°C à 200°C.

11. Procédé selon l'une des revendications 1 à 10, caractérisé en ce que le chlorure de crotyle est formé in situ à partir du butadiène présent et d'acide chlorhydrique.

12. Procédé selon l'une des revendications 1 à 11, caractérisé en ce que le complexe pi-crotyl-palladium est formé in situ à partir du butadiène présent et d'un sel du palladium soluble dans le milieu réactionnel.

13. Procédé selon l'une des revendications 1 à 12, caractérisé en ce que la concentration en eau dans le mélange réactionnel est maintenue à une valeur égale ou inférieure à 8 % et de préférence égale ou inférieure à 5 % en poids par rapport au poids dudit mélange réactionnel.

14. Procédé selon l'une des revendications 1 à 13, caractérisé en ce que le rapport molaire Cl/Pd global est inférieur ou égal à 100 et de préférence inférieur ou égal à 20.

## Claims

1. Process for the hydroxycarbonylation of butadiene or of one of its derivatives by carbon monoxide and water, at a pressure greater than atmospheric pressure and in the presence of a palladium catalyst which is soluble in the reaction medium, characterized in that:
- the process is performed in the presence of crotyl chloride, in an amount of at least two mol per mole of palladium,
- the palladium is, at least partly, in the form of a crotyl pi-complex,
- the water present in the reaction medium represents an amount less than or equal to 20% by weight relative to the weight of the reaction mixture.

2. Process according to Claim 1, characterized in that in that the total pressure at the reaction temperature is from 50 bar to 500 bar and preferably from 100 bar to 300 bar.

3. Process according to either of Claims 1 and 2, characterized in that the partial pressure of carbon monoxide, measured at 25°C, is from 25 bar to 440 bar and preferably from 55 bar to 240 bar.

4. Process according to one of Claims 1 to 3, characterized in that the amount of palladium represents from 10⁻⁵ mol to 0.2 mol per mole of butadiene employed and preferably from 10⁻⁴ mol to 0.1 mol per mole.

5. Process according to one of Claims 1 to 4, characterized in that the crotyl chloride represents, in moles, from 5 to 10 times the molar amount of palladium in the reaction medium.

6. Process according to one of Claims 1 to 5, characterized in that the butadiene concentration is at least 0.2% by weight relative to the weight of the reaction medium and preferably at least 0.5%.

7. Process according to one of Claims 1 to 6, characterized in that the butadiene concentration is less than or equal to 50% by weight relative to the weight of the reaction medium and preferably less than or equal to 30% when the process is implemented discontinuously.

8. Process according to one of Claims 1 to 6, characterized in that the butadiene concentration is less than or equal to 10% by weight relative to the weight of the reaction medium when the process is implemented continuously.

9. Process according to one of Claims 1 to 8, characterized in that the hydroxycarbonylation reaction is carried out in 3-pentenoic acid.

10. Process according to one of Claims 1 to 9, characterized in that the hydroxycarbonylation reaction is carried out at a temperature of 60°C to 230°C and preferably of 90°C to 200°C.

11. Process according to one of Claims 1 to 10, characterized in that the crotyl chloride is formed in situ from the butadiene present and hydrochloric acid.

12. Process according to one of Claims 1 to 11, characterized in that the crotyl-palladium pi-complex is formed in situ from the butadiene present and a palladium salt which is soluble in the reaction medium.

13. Process according to one of Claims 1 to 12, characterized in that the water concentration in the reaction mixture is maintained at a value less than or equal to 8% and preferably less than or equal to 5% by weight relative to the weight of the said reaction mixture.

14. Process according to one of Claims 1 to 13, characterized in that the overall Cl/Pd molar ratio is less than or equal to 100 and preferably less than or equal to 20.

## Patentansprüche

1. Verfahren zur Hydroxycarbonylierung von Butadien oder von einem seiner Derivate durch Kohlenmonoxid und Wasser bei einem Druck von höher als dem atmosphärischen Druck und in Anwesenheit eines Palladium-Katalysators, der in dem Reaktionsmedium löslich ist, dadurch gekennzeichnet, daß
- man in Anwesenheit von Crotylchlorid mit einem Verhältnis von mindestens zwei Molen pro Mol Palladium arbeitet,
- das Palladium mindestens teilweise in Form des pi-Crotyl-Komplexes vorliegt,
- das in dem Reaktionsmedium anwesende Wasser eine Menge von niedriger oder gleich 20 Gew.-% im Verhaltnis zum Gewicht der Reaktionsmischung darstellt.

2. Verfahren nach Anspruch 1, dadurch gekennzeichnet, daß der Gesamtdruck in Temperatur 50 bar bis 500 bar und vorzugsweise 100 bar bis 300 bar beträgt.

3. Verfahren nach einem der Ansprüche 1 oder 2, dadurch gekennzeichnet, daß der Partialdruck des Kohlenmonoxids, gemessen bei 25 °C, 25 bar bis 440 bar und vorzugsweise 55 bar bis 240 bar beträgt.

4. Verfahren nach einem der Ansprüche 1 bis 3, dadurch gekennzeichnet, daß die Menge an Palladium 10⁻⁵ Mol bis 0,2 Mol pro Mol eingesetztem Butadien und vorzugsweise 10⁻⁴ Mol bis 0,1 Mol pro Mol darstellt.

5. Verfahren nach einem der Ansprüche 1 bis 4, dadurch gekennzeichnet, daß das Crotylchlorid in Molen 5 bis 10 mal der molaren Menge des Palladiums in dem Reaktionsmedium darstellt.

6. Verfahren nach einem der Ansprüche 1 bis 5, dadurch gekennzeichnet, daß die Konzentration an Butadien mindestens 0,2 Gew.-% im Verhältnis zum Gewicht des Reaktionsmediums und vorzugsweise mindestens 0,5 % darstellt.

7. Verfahren nach einem der Ansprüche 1 bis 6, dadurch gekennzeichnet, daß die Konzentration an Butadien gleich oder weniger als 50 Gew.-% im Verhältnis zum Gewicht des Reaktionsmediums und vorzugsweise gleich oder weniger als 30 % beträgt, wenn das Verfahren in diskontinuierlicher Weise durchgeführt wird.

8. Verfahren nach einem der Ansprüche 1 bis 6, dadurch gekennzeichnet, daß die Konzentration an Butadien gleich oder weniger als 10 Gew.-% im Verhältnis zum Gewicht des Reaktionsmediums beträgt, wenn das Verfahren in kontinuierlicher Weise durchgeführt wird.

9. Verfahren nach einem der Ansprüche 1 bis 8, dadurch gekennzeichnet, daß die Reaktion der Hydroxycarbonylierung in Penten-3-Säure durchgeführt wird.

10. Verfahren nach einem der Ansprüche 1 bis 9, dadurch gekennzeichnet, daß die Reaktion der Hydroxycarbonylierung bei einer Temperatur von 60 °C bis 230 °C und vorzugsweise von 90 °C bis 200 °C durchgeführt wird.

11. Verfahren nach einem der Ansprüche 1 bis 10, dadurch gekennzeichnet, daß das Crotylchlorid in situ ausgehend vom anwesenden Butadien und Chlorwasserstoffsäure gebildet wird.

12. Verfahren nach einem der Ansprüche 1 bis 11, dadurch gekennzeichnet, daß der pi-Crotyl-Palladium-Komplex in situ ausgehend vom anwesenden Butadien und einem in dem Reaktionsmedium löslichen Palladiumsalz gebildet wird.

13. Verfahren nach einem der Ansprüche 1 bis 12, dadurch gekennzeichnet, daß die Konzentration an Wasser in der Reaktionsmischung auf einem Wert von niedriger oder gleich 8 Gew.-% und vorzugsweise von niedriger oder gleich 5 Gew.-% im Verhältnis zum Gewicht der genannten Reaktionsmischung gehalten wird.

14. Verfahren nach einem der Ansprüche 1 bis 13, dadurch gekennzeichnet, daß das molare Gesamtverhältnis Cl/Pd niedriger oder gleich 100 und vorzugsweise niedriger oder gleich 20 beträgt.
